(19) Europäisches Patentamt
European Patent Office
Office européen des brevets

(11) **EP 4 760 732 A1**

(12) **EUROPEAN PATENT APPLICATION**

(43) Date of publication:
17.06.2026 Bulletin 2026/25

(21) Application number: 25221371.5

(22) Date of filing: 08.12.2025

(51) International Patent Classification (IPC):
**G16H 10/40** (2018.01)   **G16H 10/60** (2018.01)
**G16H 15/00** (2018.01)

(52) Cooperative Patent Classification (CPC):
**G16H 10/40; G16H 10/60; G16H 15/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **12.12.2024 CN 202411832576**

(71) Applicant: **GE Precision Healthcare LLC
Waukesha, WI 53188 (US)**

(72) Inventors:
• **BAO, Keyu
Waukesha, 53188 (US)**

• **WANG, Hua Yi
Waukesha, 53188 (US)**
• **MEI, Haibo
Waukesha, 53188 (US)**
• **ZHAO, Dongsheng
Waukesha, 53188 (US)**
• **LIU, Jiaying
Waukesha, 53188 (US)**

(74) Representative: **Kilburn & Strode LLP
Lacon London
84 Theobalds Road
Holborn
London WC1X 8NL (GB)**

(54) **DATA PLAYBACK METHOD AND SYSTEM, AND STORAGE MEDIUM**

(57) The present disclosure relates to the field of medical data management, and in particular to a data playback method and system, and a storage medium. The method includes: acquiring medical data collected by a plurality of medical devices, wherein the medical data includes physiological parameter data of a target subject within a period of time and a medical event related to the target subject; preprocessing the physiological parameter data, wherein the preprocessing comprises: dividing the physiological parameter data into a plurality of data segments in chronological order; in response to the selection of a first time period, preloading a data segment corresponding to the first time period, and caching the preloaded data segment into a data pool; and rendering the preloaded data segment to obtain a waveform graph of the first time period; and concurrently displaying the waveform graph of the first time period and the medical event . By means of a preloading and caching mechanism, embodiments of the present disclosure implement rapid playback and display of medical data collected by a plurality of medical devices within a first time period.

FIG. 1

EP 4 760 732 A1

## Description

### TECHNICAL FIELD

The present disclosure relates to the field of medical data management, and in particular to a data playback method and system, and a storage medium.

### BACKGROUND

In modern medical practice, widespread use of medical devices has significantly improved the efficiency of diagnosis and treatment. Technological advances have enabled these devices to collect an increasing amount of physiological parameter data, which is crucial for doctors to perform disease analysis and make treatment decisions.

However, current medical data collection and playback technologies have several deficiencies. For example, the process of collecting and processing a large amount of medical data by different medical devices is not only time-consuming, but also makes effective interaction between data sources difficult to implement, which is disadvantageous for doctors. Therefore, current data playback methods usually lack interactivity and efficiency, failing to meet the needs of medical personnel for data viewing and deep analysis. These limitations restrict utilization efficiency of medical data.

### SUMMARY OF THE INVENTION

In view of this, the present disclosure provides a data playback method and system, and a storage medium.

According to one aspect of the present disclosure, a data playback method is provided. The method comprises:

acquiring medical data collected by a plurality of medical devices, wherein the medical data comprises physiological parameter data of a target subject within a period of time and a medical event related to the target subject;

preprocessing the physiological parameter data, wherein the preprocessing comprises:

dividing the physiological parameter data into a plurality of data segments in chronological order;

in response to the selection of a first time period, preloading a data segment corresponding to the first time period, and caching the preloaded data segment into a data pool; and

rendering the preloaded data segment to obtain a waveform graph of the first time period; and

concurrently displaying the waveform graph of the first time period and the medical event.

In this implementation, the medical data (comprising the physiological parameter data and the medical event) collected by the plurality of medical devices is acquired, and then the physiological parameter data is preprocessed and divided into the plurality of data segments in chronological order. By means of a preloading and caching mechanism, rapid playback and display of the physiological parameter data within the first time period are implemented. In addition, the related medical event can be presented, thereby enhancing the interactivity and efficiency of data playback, and meeting the needs of medical personnel for data viewing and deep analysis. The waveform graph of the first time period and the medical event are concurrently displayed, so that the medical personnel can more accurately understand the condition of a patient, thereby making more accurate treatment decisions, and improving utilization efficiency of the medical data and accuracy of the medical decisions. The proposal of this method is expected to improve an existing medical data management process and provide a more convenient and efficient data playback tool for the medical personnel.

In a possible implementation, the method further comprises:

in response to a user interaction request, pausing preloading of the data segment; and/or,

in response to the first time period being changed, clearing data currently cached in the data pool.

In this implementation, by pausing the preloading of the data segment in response to the user interaction request, network bandwidth and server resources can be saved, thereby improving response efficiency of user interaction. In addition, by clearing the data currently cached in the data pool in response to the first time period being changed, real-time performance and accuracy of the data are ensured.

In another possible implementation, the method further comprises:

in response to a slider of the waveform graph being slid, calculating a first moment, wherein the slider is configured to slidably adjust a displayed time range of the waveform graph, and the first moment is the time center of the adjusted waveform graph;

if the first moment indicates that the preloaded data segment is within a first time buffer zone, preloading a next data segment; and

if the first moment indicates that the preloaded data segment is not within the first time buffer zone, preloading next n data segments, wherein n is a positive integer greater than 1.

In this implementation, in response to a user operation (sliding the slider), the time center (the first moment) of the adjusted waveform graph is calculated, and the data is intelligently preloaded according to the first moment, that is, one or more data segments are preloaded according to actual needs, which improves system flexibility and scalability.

In another possible implementation, the method further comprises:

in response to a button on the waveform graph being clicked, calculating a second moment, wherein the button is configured to adjust a displayed time range of the waveform graph at fixed time intervals, and the second moment is the time center of the adjusted waveform graph;

if the second moment indicates that the preloaded data segment is within a second time buffer zone, skipping preloading; and

if the second moment indicates that the preloaded data segment is not within the second time buffer zone, preloading a next data segment.

In this implementation, in response to a user operation (clicking the button), the time center (the second moment) of the adjusted waveform graph is calculated, and the data is intelligently preloaded according to the second moment, that is, one data segment is not preloaded or preloaded according to actual needs, which improves system flexibility and scalability, prevents unnecessary data loading, and improves data loading efficiency.

In another possible implementation, the method further comprises:

in response to a time period to be played back being selected, concurrently displaying the medical events within the selected time period; and

in response to any medical event among the medical events being selected, displaying in a linked manner data of the plurality of medical devices corresponding to the selected medical event.

In this implementation, a possible linked display manner is provided, that is, event-triggered linkage: If a user selects any medical event, the system automatically positions and triggers linked display of other display positions (such as a trend chart, a waveform graph, and a parameter list) in response to the medical event being selected.

In another possible implementation, the physiological parameter data comprises a plurality of integrated parameters, and a display form of the plurality of integrated parameters further comprises at least one of a trend chart and a parameter list, the trend chart being configured to display a trend of a first set of physiological parameters of the target subject varying over time within a second time period, the waveform graph being configured to display a waveform of a second set of physiological parameters of the target subject varying over time within the first time period, the parameter list being configured to display a third set of physiological parameters of the target subject at a target moment, the first time period being contained within the second time period, and the target moment being a time point within the second time period.

In this implementation, by integrating the plurality of physiological parameters and displaying same on the same screen in different forms (the trend chart, the waveform graph, and the parameter list), a comprehensive perspective is provided to observe a patient's health state.

In another possible implementation, the method further comprises:
in response to the second time period being changed, filtering a plurality of first candidate physiological parameters that have data within the changed second time period, wherein the plurality of first candidate physiological parameters are used as a selection range for customizing the first set of physiological parameters.

In this implementation, a user is allowed to change the time period as needed, and dynamically filter valid physiological parameter data within the time period, thereby ensuring the integrity and reliability of the selected parameters, and improving system flexibility and adaptability.

In another possible implementation, the method further comprises:

in response to a first selection component being triggered, displaying the first selection component on a current interface in an overlaid manner, wherein a plurality of first candidate physiological parameters are displayed in the first selection component, and states of a first candidate physiological parameter comprise a selected state or an unselected state; and

after parameter selection is completed, using one or more of the first candidate physiological parameters in the selected state as the first set of physiological parameters, and displaying the corresponding trend chart.

In this implementation, a user can directly view all candidate physiological parameters of the trend chart on the interface, and select parameters of interest as needed. Such an intuitive selection manner improves the user's operation efficiency. In addition, the user is allowed to select parameters according to personal needs, so that presentation of the trend chart is more personalized, meeting specific needs of different users for data display.

In another possible implementation, the method further comprises:

in response to any position in the trend chart being selected, displaying in a linked manner the waveform graph and/or the parameter list corresponding to a third moment, wherein the third moment is a time point corresponding to the selected position in the trend chart.

In this implementation, another possible linked display manner is provided, that is, trend chart-triggered linkage: If a user selects any position in the trend chart, the system automatically positions and triggers linked display of other display positions (such as the waveform graph and the parameter list) in response to any position in the trend chart being selected.

In another possible implementation, the method further comprises:

in response to any position in the trend chart being selected, displaying in the form of a pop-up box information of the first set of physiological parameters corresponding to the third moment.

In this implementation, when a user selects any position in the trend chart, the system can immediately provide physiological parameter information at the corresponding moment in the form of a pop-up box, which enhances real-time performance and interactivity of the information.

In another possible implementation, the method further comprises:

in response to a second selection component being triggered, displaying the second selection component on a current interface in an overlaid manner, wherein a plurality of second candidate physiological parameters are displayed in the second selection component, and states of a second candidate physiological parameter comprise a selected state or an unselected state; and

after parameter selection is completed, using one or more of the second candidate physiological parameters in the selected state as the second set of physiological parameters, and displaying the corresponding waveform graph.

In this implementation, a user can directly view all candidate physiological parameters of the waveform graph on the interface, and select parameters of interest as needed. Such an intuitive selection manner improves the user's operation efficiency. In addition, the user is allowed to select parameters according to personal needs, so that presentation of the waveform graph is more personalized, meeting specific needs of different users for data display.

In another possible implementation, the method further comprises:

in response to a start moment being selected, displaying the corresponding waveform graph using the start moment as the time center of the waveform graph, wherein the time center represents a midpoint of a displayed time range of the waveform graph; or,

in response to any medical event among the medical events being selected, displaying the corresponding waveform graph using a start moment of the selected medical event as the time center of the waveform graph; or,

in response to any position in the trend chart being selected, displaying the corresponding waveform graph using a moment corresponding to the selected position as the time center of the waveform graph.

In this implementation, the system can respond to various user operations, comprising selecting the start moment, selecting the medical event, or selecting any position in the trend chart, and display the corresponding waveform graph using a time point corresponding to the operation as the time center of the waveform graph, ensuring that the user can view physiological parameter variations around the time center.

In another possible implementation, the method further comprises:

in response to a target control on the waveform graph being triggered, displaying the adjusted waveform graph, and displaying in a linked manner the parameter list corresponding to a fourth moment;

wherein the target control is configured to adjust a displayed time range of the waveform graph, and the fourth moment is the time center of the adjusted waveform graph.

In this implementation, another possible linked display manner is provided, that is, waveform graph-triggered linkage: If a user triggers a target control (such as an operation of clicking the button or sliding the slider) on the waveform graph, the system automatically positions and triggers linked display of other display positions (such as the parameter list) in response to the target control on the waveform graph being triggered.

According to another aspect of the present disclosure, a data playback system is provided. The system comprises:

a processor;

a display, configured to receive an instruction from the processor to perform display; and

a memory, configured to store instructions executable by the processor, wherein

the processor is configured to implement the above method when executing the instructions stored in the memory.

In a possible implementation, the plurality of medical devices comprise a plurality of devices selected from a group comprising a syringe pump, a monitor, an electrocardiograph, an electroencephalograph, a ventilator, and an anesthesia machine.

In another possible implementation, the data playback system is a system independent of the plurality of medical devices, the data playback system performs data exchange with the plurality of medical devices by means of a server, and the server is configured to integrate and process data collected by the plurality of medical devices.

According to another aspect of the present disclosure, a non-volatile computer-readable storage medium is provided, having computer program instructions stored thereon, wherein the computer program instructions, when executed by a processor, implement the above method.

According to another aspect of the present disclosure, a computer program product is provided, comprising computer-readable code or a non-volatile computer-readable storage medium bearing computer-readable code, wherein when the computer-readable code is run in a processor of a computing device, the processor in the computing device executes the above method.

Other features and aspects of the present disclosure will become apparent from the following detailed description of exemplary embodiments provided with reference to the accompanying drawings.

BRIEF DESCRIPTION OF THE DRAWINGS

The accompanying drawings, incorporated in and constituting a part of the specification, illustrate exemplary embodiments, features, and aspects of the present disclosure together with the specification, and serve to explain the principles of the present disclosure.

FIG. 1 shows a schematic diagram of a structure of a data playback scenario provided by an exemplary embodiment of the present disclosure.

FIG. 2 shows a flowchart of a data playback method provided by an exemplary embodiment of the present disclosure.

FIG. 3 shows a schematic diagram of an interface of a data playback system provided by an exemplary embodiment of the present disclosure.

FIG. 4 shows a schematic diagram of a principle of linked display provided by an exemplary embodiment of the present disclosure.

FIG. 5 shows a schematic diagram of a principle of linked display provided by another exemplary embodiment of the present disclosure.

FIG. 6 shows a flowchart of a data playback method provided by another exemplary embodiment of the present disclosure.

FIG. 7 shows a schematic diagram of an interface for event-triggered linkage provided by an exemplary embodiment of the present disclosure.

FIG. 8 shows a schematic diagram of an interface of a first selection component provided by an exemplary embodiment of the present disclosure.

FIG. 9 shows a schematic diagram of an interface for trend chart-triggered linkage provided by an exemplary embodiment of the present disclosure.

FIG. 10 shows a schematic diagram of an interface of a second selection component provided by an exemplary embodiment of the present disclosure.

FIG. 11 shows a schematic diagram of an interface for waveform graph-triggered linkage provided by an exemplary embodiment of the present disclosure.

FIG. 12 shows a flowchart of a data preloading process of a waveform graph provided by an exemplary embodiment of the present disclosure.

FIG. 13 shows a flowchart of a segment loading solution for a waveform graph provided by an exemplary embodiment of the present disclosure.

FIG. 14 is a block diagram of an apparatus according to an exemplary embodiment.

DETAILED DESCRIPTION

Various exemplary embodiments, features, and aspects of the present disclosure will be described in detail below with reference to the accompanying drawings. The same reference numerals in the drawings represent elements having the same or similar functions. While various aspects of the embodiments are illustrated in the accompanying drawing, the accompanying drawings are not necessarily drawn to scale unless specifically stated.

The word "exemplary" dedicated herein means "used as an example or an embodiment, or is illustrative". Any embodiment illustrated herein as "exemplary" is not necessarily interpreted as being superior to or better than other embodiments.

In addition, in order to better illustrate the present disclosure, numerous specific details are given in the detailed description below. It should be understood by those skilled in the art that the present disclosure can be implemented without some of the specific details. In some examples, the methods, means, elements, and circuits well known to those skilled in the art are not described in detail in order to highlight the main idea of the present disclosure.

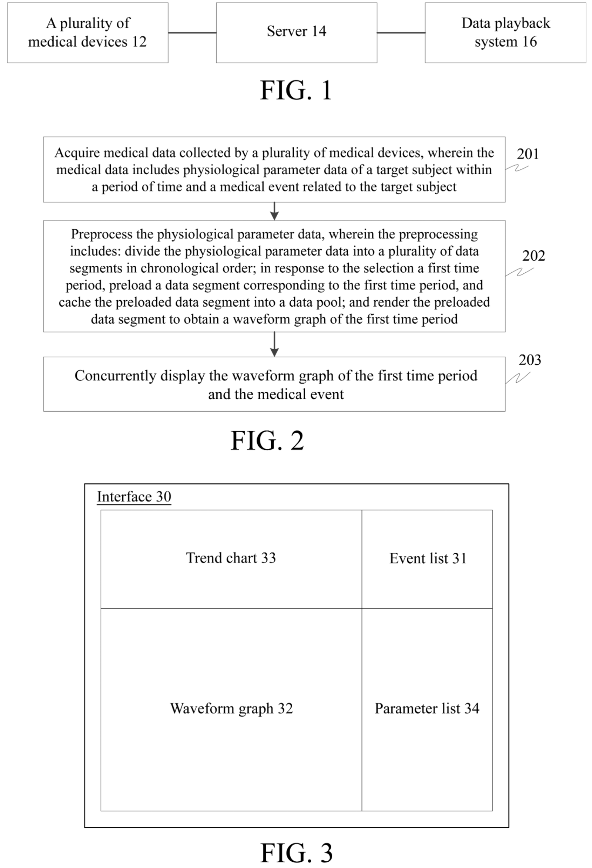

Embodiments of the present disclosure provide a data playback system. The system is particularly applicable to the medical field, especially in a scenario where physiological parameter data of patients and medical events need to be monitored and played back. For example, the system may be used in an operating room, an intensive care unit (ICU), or an emergency room. Refer to FIG. 1, which shows a schematic diagram of a structure of a data playback scenario provided by an exemplary embodiment of the present disclosure. The data playback scenario may include a plurality of medical devices 12, a server 14, and a data playback system 16.

The medical device 12 refers to an instrument, a device, an appliance, a material, or the like for medical purposes, including required computer software. The plurality of medical devices 12 may include a plurality of devices selected from a group including a syringe pump, a monitor, an electrocardiograph, an electroencephalograph, a ventilator, and an anesthesia machine.

The plurality of medical devices 12 collect patient data by means of respective sensors and data collection systems. In some embodiments, the data collected by the plurality of medical devices 12 may include the following content: 1. Syringe pump: The syringe pump is mainly configured to accurately control an injection rate and an injection dosage of medication, and data collected by the syringe pump usually includes information such as the name of the injected medication, the injection rate, and the injection dosage. 2. Monitor: The monitor is configured to monitor a patient's vital signs in real time, and data collected by the monitor includes body temperature, heart rate, blood pressure, peripheral capillary oxygen saturation (SpO2), and the like. 3. Electrocardiograph: The electrocardiograph records an electrical activity of the heart, and data collected by the electrocardiograph includes electrocardiogram waveform data, which can be used to analyze a rhythm and a functional state of the heart. 4. Electroencephalograph: The electroencephalograph measures an electrical activity of the brain, and data collected by the electroencephalograph includes electroencephalogram waveform data, which can be used to diagnose neurological diseases such as epilepsy and sleep disorders. 5. Ventilator: The ventilator is configured to assist a patient's breathing, and data collected by the ventilator includes tidal volume, inspiratory pressure, expiratory pressure, respiratory rate, oxygen concentration, and the like. 6. Anesthesia machine: The anesthesia machine is configured to provide anesthesia during surgery, and data collected by the anesthesia machine may include the concentration of an inhaled anesthetic, oxygen concentration, respiratory parameters, and the like. The above is not limited in embodiments of the present disclosure.

The data playback system 16 performs data exchange with these medical devices 12 by means of the server 14. The server 14 serves as a central node and is responsible for centralized integration processing of the dispersed data collected by the plurality of medical devices 12. That is, a main function of the server 14 is to integrate these data from different medical devices 12 to form comprehensive and continuous medical data. This step involves unifying a format of the dispersed data, synchronizing timestamps, and ensuring consistency and accuracy of the data, finally forming a complete set of medical data. The medical data not only includes physiological parameter data of a patient within a period of time, such as heart rate, blood pressure, body temperature, and respiratory rate, but also includes a medical event related to the patient, such as an intraoperative alarm event. The integrated medical data is stored in the server 14 for subsequent data playback and analysis.

By means of integrated processing of the server 14, the medical data becomes more orderly and easy to analyze, providing a unified view for medical professionals to monitor and manage a patient's health state. Such data integration not only improves data management efficiency, but also enhances availability and accessibility of the data, making medical decisions more accurate and prompt. In addition, the server 14 may further be responsible for data backup, secure storage, and privacy protection, ensuring integrity and compliance of the medical data.

The data playback system 16 acquires the integrated medical data from the server 14, and performs data reproduction and analysis to support medical decision-making and patient monitoring. The data playback system 16 is a system independent of the plurality of medical devices 12, and is configured to execute a data playback method provided by embodiments of the present disclosure. The data playback method is intended to comprehensively capture and reproduce key data in a medical monitoring or treatment process, especially for a patient who is undergoing medical monitoring or treatment.

In some embodiments, the data playback system 16 may include a processor, a display, and a memory, wherein the display is configured to receive instructions from the processor to perform display. The memory is configured to store instructions executable by the processor. The processor is configured to implement the data playback method when executing the instructions stored in the memory. For ease of explanation, the data playback system 16 may be referred to as the system hereinafter.

Main steps and strategies of the data playback method are as follows: 1. Data collection and integration: The system comprehensively collects data from different medical devices 12 aggregated by means of the server 14, including an alarm event of an intraoperative patient, and data from devices such as the syringe pump, the monitor, and the anesthesia machine, ensuring data integrity and continuity. 2. Data preprocessing and presentation: The physiological parameter data is preprocessed and divided into a plurality of data segments in chronological order for ease of management and analysis. In response to a user selection, the data segment for the corresponding time period is preloaded and cached into a data pool to improve a data access speed and efficiency. The preloaded data segment is rendered to generate a waveform graph, and the waveform graph and the medical event of the time period are concurrently displayed, providing the user with an intuitive data view. 3. Data linkage and positioning: The system implements automatic association of the data, and when the user clicks any event in an event list of the intraoperative patient, the system automatically positions the data of the medical devices such as the syringe pump, the monitor, the anesthesia machine. and the like at the same moment based on an occurrence time of the event. A linkage sequence may start from the event list, to a trend chart, the waveform graph, and a parameter list, ensuring that the user can quickly and accurately acquire required information. 4. Parameter selection and optimization: Considering a huge amount of data generated by the intraoperative devices, the system automatically filters parameters that have data within the time period selected by the user, providing flexible parameter selection and matching. 5. Performance improvement: In combination with an algorithm and a preloading concept, the system significantly shortens time for data loading, presentation, and interaction, improving user experience. In summary, the data playback system 16 having characteristics of high efficiency, intuition, and intelligence provides a powerful tool for medical data management and analysis, greatly improving the quality and efficiency of medical services.

The data playback method provided by the embodiments of the present disclosure is introduced below with reference to several exemplary embodiments.

Refer to FIG. 2, which shows a flowchart of a data playback method provided by an exemplary embodiment of the present disclosure. This embodiment is illustrated by taking the method being used in the above data playback system as an example. The method includes the following steps:

Step 201: Acquire medical data collected by a plurality of medical devices, wherein the medical data includes physiological parameter data of a target subject within a period of time and a medical event related to the target subject.

The data playback system performs data exchange with the plurality of medical devices by means of a server, to acquire medical data collected by the plurality of medical devices. In some embodiments, the plurality of medical devices collect data of the target subject by means of respective sensors and data collection systems. The server integrates the data collected by the plurality of medical devices to obtain medical data, and the integrated medical data is stored in the server. The data playback system obtains the integrated medical data from the server.

The medical data includes not only physiological parameter data of the target subject within a certain period of time, but also covers the medical event related to the target subject. The target subject generally refers to a patient undergoing medical monitoring or treatment.

The physiological parameter data is collected and integrated by the plurality of medical devices, that is, the physiological parameter data includes a plurality of integrated parameters. The physiological parameter data includes, but is not limited to, key vital signs such as heart rate, blood pressure, body temperature, and peripheral capillary oxygen saturation. The above is not limited in embodiments of the present disclosure.

The medical events refers to any significant medical condition that occurs while the target subject is receiving medical services. These events may significantly impact health and treatment outcomes of the target subject. The medical events include, but are not limited to: 1. Physiological parameter abnormality: A significant variation in any physiological parameter of the target subject during monitoring, such as the heart rate, the blood pressure, or the peripheral capillary oxygen saturation exceeding a normal range. 2. Intraoperative alarm event: An alarm triggered during surgery due to a sudden variation in a physiological state of the target subject or an abnormal situation detected by the device. 3. Treatment response: A response of the target subject to a specific treatment, including effects and side effects of medication, surgical operation, or other treatment measures. The above is not limited in embodiments of the present disclosure.

Step 202: Preprocess the physiological parameter data. The preprocessing includes: dividing the physiological parameter data into a plurality of data segments in chronological order; in response to the selection a first time period, preloading a data segment corresponding to the first time period, and caching the preloaded data segment into a data pool; and rendering the preloaded data segment to obtain a waveform graph of the first time period.

In some embodiments, the data playback system preprocesses the physiological parameter data, including but not limited to the following steps:

1. Data segment division: First, the collected physiological parameter data is divided into the plurality of data segments in chronological order. A data segment refers to an independent part or fragment formed by partitioning the continuous physiological parameter data in chronological order. This step is to partition a continuous data stream into manageable data blocks for subsequent processing and analysis.

2. Data preloading: In response to the selection the first time period, the system preloads the data segment corresponding to the first time period. This means that the system reads and prepares the data in advance that the user is going to view or analyze, to improve the efficiency and response speed of data processing.

3. Data caching: The preloaded data segment is cached into the data pool. The data pool is a temporary storage region for storing preloaded data for quick access and processing.

4. Data rendering: The preloaded data segment is rendered, that is, the original physiological parameter data is converted into a visualized waveform graph. The waveform graph is a graphical representation method for presenting physiological parameter data varying over time, usually presented in the form of a waveform. This step involves a graphical representation of the data, so that a medical professional can intuitively observe and analyze physiological parameter variations of the patient.

It should be noted that for details of a segment loading solution of the waveform graph, reference may be made to related description in the following embodiments, which will not be introduced here.

Step 203: Concurrently display the waveform graph of the first time period and the medical event.

In some embodiments, the data playback system concurrently displays, in the form of a dashboard (English: dashboard), the waveform graph of the first time period and the medical event on the same interface. The waveform graph is configured to display a waveform of a set of physiological parameters of the target subject varying over time within a first time period. The medical event includes one or more medical events of the target subject within a third time period, and the first time period is contained within the third time period.

In some embodiments, the waveform graph and the medical event are concurrently displayed, and a plurality of display manners may be used to ensure information clarity and legibility. The following are some possible display manners: 1. Upper-lower layout: The waveform graph is displayed in the upper half of the interface and the medical event is displayed in the lower half, which can maintain vertical separation of the content. 2. Left-right layout: The waveform graph is displayed on the left side of the interface and the medical event is displayed on the right side, which can maintain horizontal separation of the content. 3. Overlaid display: The waveform graph is used as a background, and the medical event is overlaid on the waveform graph in the form of an annotation, highlight, or another visual element. However, it is ensured that a main part of the waveform graph is not obscured. 4. Split view: Two independent view regions are provided in the same window. The user can adjust display region sizes of the waveform graph and the medical event by dragging a separation bar. In addition, when the user selects or views a specific medical event, the view of the waveform graph is also dynamically updated to reflect the time period of the current medical event. The above is not limited in embodiments of the present disclosure.

In summary, the embodiments of the present disclosure provide a data playback method. According to the method, medical data (including physiological parameter data and a medical event) collected by a plurality of medical devices is acquired, and then the physiological parameter data is preprocessed and divided into a plurality of data segments in chronological order. By means of a preloading and caching mechanism, rapid playback and display of data within a first time period are implemented, and a related medical event can be concurrently presented, thereby enhancing data interactivity and accuracy, and meeting the needs of medical personnel for data viewing and deep analysis. Especially for an application scenario involving a large amount of medical data from a plurality of medical devices, the preloading and caching mechanism of the embodiments of the present disclosure can ensure the immediacy of a system response when the medical personnel view data, and reduce delays caused by data processing. Further, by concurrently displaying a waveform graph of the first time period and a medical event, the medical personnel can more accurately understand a patient's condition, thereby making more accurate treatment decisions, and improving utilization efficiency of the medical data and accuracy of the medical decisions. The proposal of this method is expected to improve an existing medical data management process and provide a more convenient and efficient data playback tool for the medical personnel.

In some embodiments, a display form of the physiological parameter data is not limited to the waveform graph, and may further include at least one of a trend chart and a parameter list, to provide more comprehensive data analysis. The trend chart is configured to display a trend of a first set of physiological parameters of a target subject varying over time within a second time period. The waveform graph is configured to display a waveform of a second set of physiological parameters of the target subject varying over time within the first time period. The parameter list is configured to display a third set of physiological parameters of the target subject at a target moment. The first time period is contained within the second time period. The target moment is a time point within the second time period.

That is, the trend chart is generally configured to present an overall variation trend of the physiological parameters (the first set of physiological parameters) of the target subject varying over time within a longer time period (the second time period). The first set of physiological parameters may include heart rate, blood pressure, body temperature, respiratory rate, and the like. Data in the trend chart is typically aggregated, meaning that the data may be an average or summary data over a time interval, to facilitate observation of the overall trend and pattern. The trend chart emphasizes continuity and smoothness of the data. A continuous curve is formed by connecting data points to present smooth variations of the parameters over time.

The waveform graph is generally configured to present detailed waveforms of the physiological parameters (the second set of physiological parameters) of the target subject varying over time within a shorter time period (the first time period). The second set of physiological parameters may include an electrocardiogram (ECG), an electroencephalogram (EEG), an electromyogram (EMG), and the like. The data in the waveform graph is more detailed, typically presenting raw, unaggregated data points to facilitate observation of instantaneous variations and details of the parameters. The waveform graph emphasizes accuracy and real-time performance of the data, and is appropriate for occasions that require accurate analysis and diagnosis, such as arrhythmia diagnosis.

The parameter list is usually configured to display physiological parameters (the third set of physiological parameters) of the target subject at the target moment. These parameters provide an instant snapshot related to an individual's health state. The third set of physiological parameters may include peripheral capillary oxygen saturation, respiratory rate, body temperature, and the like, which can immediately reflect a current physiological state of the patient.

Similar to the above various display manners used for concurrently displaying the waveform graph and the medical event, when in design, the system can select the most appropriate presentation manner for concurrently displaying the waveform graph, the medical event, the trend chart, and the parameter list based on the user's specific needs and preferences, or provide a plurality of display options for the user to select according to actual situations. Such a design not only improves information clarity and legibility, but also enhances user experience and system practicability.

Refer to FIG. 3, which shows a schematic diagram of an interface of a data playback system provided by an exemplary embodiment of the present disclosure. The system presents medical events in the form of an event list 31, and presents physiological parameter data in the form of a waveform graph 32, a trend chart 33, and a parameter list 34 on the same interface 30, providing a comprehensive data view.

In some embodiments, a user determines any medical event in the event list, and the system automatically positions and displays a plurality of integrated parameters of a plurality of medical devices at the same moment in a linked manner based on an occurrence time of the medical event.

Linked display is a technology commonly used in data visualization and monitoring systems, which allows a plurality of data presentation components (such as a chart and a list) to be associated and interacted with each other. The following is a specific introduction to linked display:

1. Event-triggered linkage: When the user selects or clicks a specific medical event in the event list, the system automatically positions data of other related components (the trend chart, the waveform graph, the parameter list) to a time point when the medical event occurs. In this way, medical personnel can immediately view a patient's physiological parameters and state when the event occurs, so as to quickly make a judgment and a response.

2. Trend chart-triggered linkage: When the user selects a specific time period or data point in the trend chart, the system automatically updates the waveform graph and the parameter list, and presents detailed data corresponding to a selected part of the trend chart.

3. Waveform graph-triggered linkage: A user operation (such as clicking a button and sliding a slider) that moves a displayed time range of the waveform graph also triggers linkage, so that the parameter list is synchronously updated to reflect selected or viewed data in the waveform graph.

A linkage sequence starts from the event list, to the trend chart, the waveform graph, and the parameter list, implementing rapid data positioning and presentation. This sequence ensures a data viewing process from macro to micro. The user can quickly position a specific question from the event list, then understand a detailed variation of the question by means of the trend chart and the waveform graph, and finally acquire a specific value by means of the parameter list.

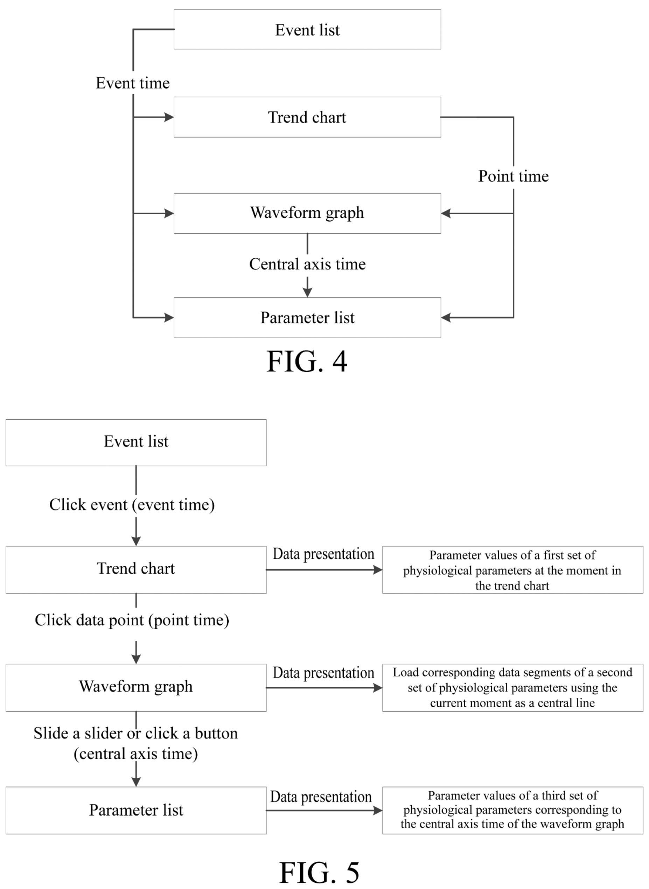

Refer to FIG. 4, which shows a schematic diagram of a principle of linked display provided by an exemplary embodiment of the present disclosure. This figure describes a linkage relationship between different data presentation components in the system. The individual components and connections therebetween are as follows:

1. Event list: When the user selects one medical event in the event list, the system updates display of the trend chart, the waveform graph, and the parameter list according to the time point (also referred to as an event time) when the medical event occurs.

2. Trend chart: When the user selects a specific time point (also referred to as a point time) in the trend chart, the system updates the waveform graph and the parameter list to display detailed data of the time point.

3. Waveform graph: The time center of the waveform graph may be synchronized with the selected time point in the trend chart to display waveform data of the corresponding time point. When the user moves the displayed time range of the waveform graph, the system determines a time center (also referred to as a central axis time) of the waveform graph after moving, and updates the corresponding parameter list.

4. Parameter list: When the user determines a time point in the event list or the trend chart or the waveform graph, the parameter list corresponding to the time point is synchronously updated.

Arrows in the figure indicate directions of data flow and linkage: The arrows from the event list to the trend chart, the waveform graph, and the parameter list indicate that selecting the medical event may update the trend chart, the waveform graph, and the parameter list. The arrows from the trend chart to the waveform graph and the parameter list indicate that selecting the time point in the trend chart may update the waveform graph and the parameter list. The arrow from the waveform graph to the parameter list indicates that moving the time center in the waveform graph may update the parameter list.

FIG. 5 further details interaction processes between the user and different data presentation components in the system, and how these interactions trigger data display and updates. Refer to FIG. 5, which shows a schematic diagram of a principle of a linked display provided by another exemplary embodiment of the present disclosure. The individual components and connections therebetween are as follows:

1. Event list: The user clicks any medical event in the event list.

2. Trend chart: After the medical event is clicked, the system detects the clicked event, updates the trend chart, the waveform graph, and the parameter list according to the event time, and presents data related to the medical event. On the trend chart, when the user clicks a data point, the system may update the waveform graph and the parameter list according to the point time, and perform data presentation, presenting parameter values of the first set of physiological parameters at the moment in the trend chart.

3. Waveform graph: The data point selected by the user on the trend chart may be transferred to the waveform graph, and the system may load corresponding data segments of the second set of physiological parameters using the current moment as a center line, so as to provide the user with deeper waveform analysis.

4. Parameter list: In addition, a user operation (such as sliding the slider or clicking the button) on the waveform graph may update the parameter list and present parameter values of the third set of physiological parameters corresponding to the central axis time of the waveform graph, further enriching understanding and analysis of the data by the user.

By means of a linked display mechanism of this design, the system not only improves intuitiveness and ease of use of data presentation, but also enhances insight of the user into the relationship between the medical events and the physiological parameters.

In summary, the embodiments of the present disclosure provide a data playback method, which further has the following beneficial effects:
The method provided by the embodiments of the present disclosure integrates device data into modules such as a trend chart, a waveform graph, and a parameter list, and implements presentation on the same screen, operations on the same screen, linkage on the same screen, and one-click positioning.

The method provided by the embodiments of the present disclosure not only collects data of a plurality of different medical devices, but also classifies and integrates the data of the different medical devices, providing diverse data to ensure doctors' post-operative scientific research.

The method provided by the embodiments of the present disclosure performs preloading, caching, and rendering according to user operations, can automatically save the user's operation habits, and further provides many one-click methods to help the doctors perform one-click positioning and one-click analysis.

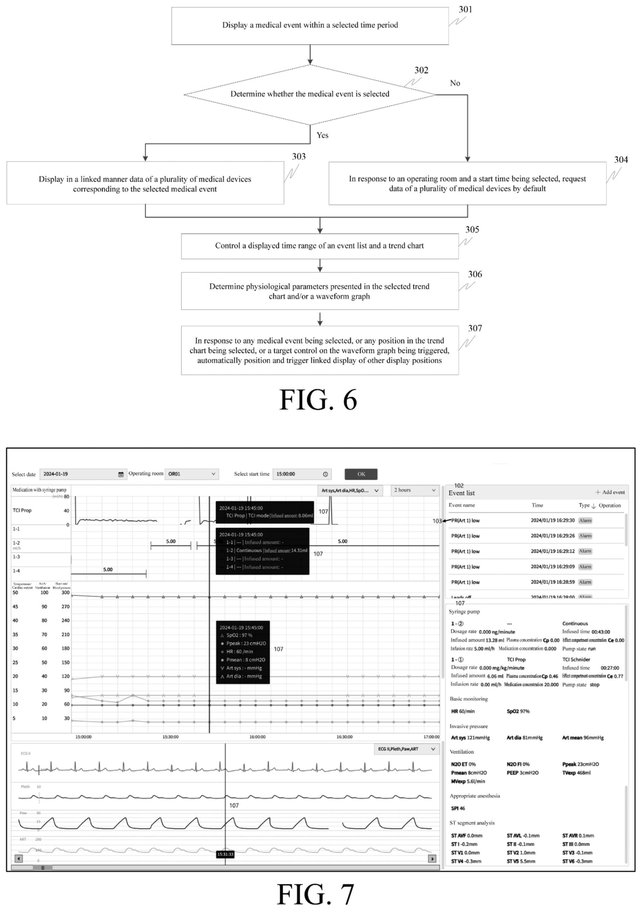

Refer to FIG. 6, which shows a flowchart of a data playback method provided by another exemplary embodiment of the present disclosure. This embodiment is illustrated by taking the method being used in the above data playback system as an example. The method includes the following steps:
Step 301: Display a medical event within a selected time period.

In some embodiments, the system displays the medical event within the selected time period, which may include the following steps: 1. Respond to selection: When the user selects a specific time period for data playback, the system may automatically recognize and respond to this selection. 2. Query events: The system automatically queries all medical events that occurred within the time period. 3. Display an event list: An event list is concurrently displayed on an interface. The event list includes one or more medical events queried within the selected time period.

In some embodiments, selection manners of the time period may include, but are not limited to, the following: 1. Manual input The user directly sets start and end times of the time period by inputting a specific date and time. 2. Selection with slider bar: A time slider bar is provided, and the user selects a time range by dragging two ends of the slider bar. 3. Selection with pull-down menu: Preset time range options are provided by a pull-down menu, such as "last 1 hour" and "last 24 hours". The user selects one option to select a time period. 4. Calendar selector: A calendar interface is provided, and the user can define a time period by selecting a specific date. 5. Operation with shortcut keys: The user may quickly jump to a specific time period by using a preset shortcut key combination, such as "last day" and "next day".

Step 302: Determine whether the medical event is selected.

The system may detect whether the user selects (such as clicks) any medical event in the event list. If the system detects that any medical event in the event list is selected, step 303 is executed. Otherwise, step 304 is executed.

Step 303: In response to any medical event among the medical events being selected, display in a linked manner data of a plurality of medical devices corresponding to the selected medical event.

If the system detects that any medical event in the event list is selected, default parameter data related to the medical event is automatically requested based on an operating room and a time of the medical event as indexes. Linkage of display positions such as the trend chart, the waveform graph, and the parameter list is triggered, to present data related to the selected medical event.

Step 304: In response to an operating room and a start time being selected, request data of the plurality of medical devices by default.

If the system does not detect that any medical event in the event list is selected, the system may respond to the operating room and the start time selected by the user. The system may request the data of the plurality of medical devices by default, so that the data can be presented without the selection of a specific medical event.

Step 305: Control a displayed time range of the event list and the trend chart.

When the user selects a specific duration, the system may control, in response to the specific duration being selected, the displayed time range of the event list and the trend chart, to ensure that the presented data matches the duration selected by the user.

Step 306: Determine physiological parameters presented in the selected trend chart and/or the waveform graph.

The system allows the user to select the physiological parameters presented in the trend chart and/or the waveform graph. According to user selection, the system accordingly updates presentation content of the trend chart and the waveform graph.

In some embodiments, the system may display a selection component on a current interface in an overlaid manner, and a plurality of candidate physiological parameters are displayed in the selection component. States of the candidate physiological parameters include a selected state or an unselected state. After parameter selection is completed, one or more candidate physiological parameters in the selected state are used as a set of physiological parameters, and the corresponding trend chart or waveform graph is displayed.

Step 307: In response to any medical event being selected, or any position in the trend chart being selected, or a target control on the waveform graph being triggered, automatically position and trigger linked display of other display positions.

In some embodiments, the method for automatically positioning and triggering linked display by the system includes, but is not limited to, the following possible implementations: 1. Event-triggered linkage: If a user selects any medical event, the system automatically positions and triggers linked display of other display positions (such as a trend chart, the waveform graph, and a parameter list) in response to the medical event being selected. 2. Trend chart-triggered linkage: If the user selects any position in the trend chart, the system also automatically positions and triggers linked display of other display positions (such as the waveform graph and the parameter list) in response to any position in the trend chart being selected. 3. Waveform graph-triggered linkage: If the user triggers a target control (such as an operation of clicking a button or sliding a slider) on the waveform graph, the system also automatically positions and triggers linked display of other display positions (such as the parameter list) in response to the target control on the waveform graph being triggered.

The above steps jointly constitute a complete operation process, which ensures that the user can quickly and accurately acquire and view the medical device data related to the selected time period and event when playing back the medical data. By means of such a linked display mechanism, the user can more intuitively understand the data and make prompt medical decisions.

Features of the medical event, the trend chart, and the waveform graph are described in more detail below in sequence.

The medical events are usually displayed in the form of an event list. The event list may include a plurality of rows, and each row records key information of one medical event in detail. Illustratively, each row may contain three main portions: 1. Event name: Identify the name or description of the medical event, which enables the user to quickly identify nature of the event. 2. Time: Record a specific time when the medical event occurs, which is critical for event tracking and analysis. 3. Type: Indicate classification of the medical event, which facilitates further classification and processing of the event. Classification labels may be used for types, and the labels are assigned to different types of medical events, such as "urgent", "warning", and "notification". Alternatively, the types may be color-coded. Color-coding is used to distinguish different types of events, for example, red represents emergency, and yellow represents warning, which facilitates further classification and processing of the events.

For an event list having a large number of medical events, a scroll bar may be provided to browse all the medical events. In addition, the event list may further have an interactive function. The interactive function may include one or more of the following functions: 1. Sorting function: Allow the user to sort the event list by the event name, time, or type. 2. Search function: Provide a search box, so that the user can enter a keyword to quickly find a specific medical event. 3. Filtering function: Allow the user to filter the event list based on the event type or other attributes. 4. Linked display triggering function: If the user selects any medical event, the system automatically positions and triggers linked display of other display positions (such as the trend chart, the waveform graph, and the parameter list).

Refer to FIG. 7, which shows a schematic diagram of an interface for event-triggered linkage provided by an exemplary embodiment of the present disclosure. The system displays medical events 103 in the form of an event list 102. Each row of the event list 102 represents one medical event 103. The event list 102 includes a plurality of columns, such as event name, time, and type. In response to any medical event 103 being selected, the system automatically requests data 107 of a plurality of medical devices related to the medical event 103 based on an operating room and a time of the medical event 103 as indexes, and displays the data 107 of the plurality of medical devices in a linked manner on the same interface in the form of a waveform graph, a trend chart, and a parameter list, providing a comprehensive data view.

Features of the trend chart include, but are not limited to: 1. Parameter and duration selection: The user can freely select physiological parameters and a displayed time range presented in the trend chart by means of a selection component. Such flexibility allows users to customize their trend chart views as desired. 2. Dynamic parameter filtering: When the user changes a duration option, the system automatically filters physiological parameters that have data within the time range, thereby narrowing the range of selectable physiological parameters. Such an intelligent filtering mechanism ensures that the user only selects those physiological parameters that have valid data within a specific time period, which improves selection accuracy. Since all selectable parameters exist based on actual data, the selection of parameters that have no data is prevented.

In some embodiments, the trend chart is configured to display a trend of a first set of physiological parameters of a target subject varying over time within a second time period. If the user changes the second time period, the system filters a plurality of first candidate physiological parameters that have data within the changed second time period in response to the second time period being changed. A plurality of first candidate physiological parameters are used as a selection range for customizing the first set of physiological parameters. If the user triggers a first selection component, the system, in response to the first selection component being triggered, displays the first selection component on a current interface in an overlaid manner. A plurality of first candidate physiological parameters are displayed in the first selection component. States of the first candidate physiological parameter include a selected state or an unselected state. After parameter selection is completed, one or more first candidate physiological parameters in the selected state are used as the first set of physiological parameters, and a corresponding trend chart is displayed.

Further, a parameter selection and display mechanism of the trend chart may include, but is not limited to, the following steps: 1. Current display of trend chart: The system currently displays the trend chart, presenting a trend of the first set of physiological parameters of the target subject varying over time within the second time period. This display provides the user with a basic view of a physiological state of the target subject. 2. Response to change of second time period: If the user changes the second time period, the system immediately responds to this change. The system captures variations of the second time period by monitoring a user input, and prepares for subsequent data filtering and updating operations. 3. Data filtering mechanism: The system executes a data filtering operation within the changed second time period. This operation involves retrieving relevant physiological parameter data stored in a database or data storage to determine which parameters have available data records within a new time period. 4. Determination of candidate physiological parameters: After filtering is completed, the system determines a plurality of first candidate physiological parameters that have data records within the new time period. These parameters constitute a new selection range for the customization of the first set of physiological parameters by the user, ensuring that the user only selects those parameters that have data support within the new time period. 5. Update of parameter selection range: The system updates display content of the first selection component. After the first selection component is triggered, the first selection component is displayed on a current interface in an overlaid manner. That is, the plurality of first candidate physiological parameters obtained by filtering are presented to the user. These parameters are presented in an optional form, including the selected state and the unselected state, allowing the user to select as needed. 6. Dynamic update of trend chart: After the user completes parameter selection in the updated first selection component, the system dynamically updates the trend chart based on user selection, and presents a trend of the newly selected first set of physiological parameters varying over time within the changed second time period.

The manner for changing the second time period may refer to the above manner for selecting the time period, for example, selection with a pull-down menu. Preset time range options are provided by means of a pull-down menu, such as "last 1 hour" and "last 2 hours". The user selects one option to change the second time period.

Manners for triggering the first selection component may include, but are not limited to, the following: 1. Click operation: The user triggers the first selection component by clicking a button or icon on an interface. 2. Mouse hovering: When the user's mouse hovers over a specific region, the first selection component automatically pops up and displays. 3. Right-click menu: The user selects one option from a pop-up context menu by right-clicking an element on the interface to trigger the first selection component. 4. Activation with shortcut keys: The user quickly triggers the first selection component by pressing a preset shortcut key. 5. Voice command: In a system that supports speech recognition, the user may trigger the first selection component by speaking a particular voice command. 6. Automatic triggering: In some cases, the system may automatically trigger the first selection component according to a specific logic or condition, such as when the user views a specific type of data. These manners for changing time periods and triggering components provide the user with flexible and diverse interaction means to adapt to different usage scenarios and personal preferences, thereby enhancing user experience and operation convenience of the system.

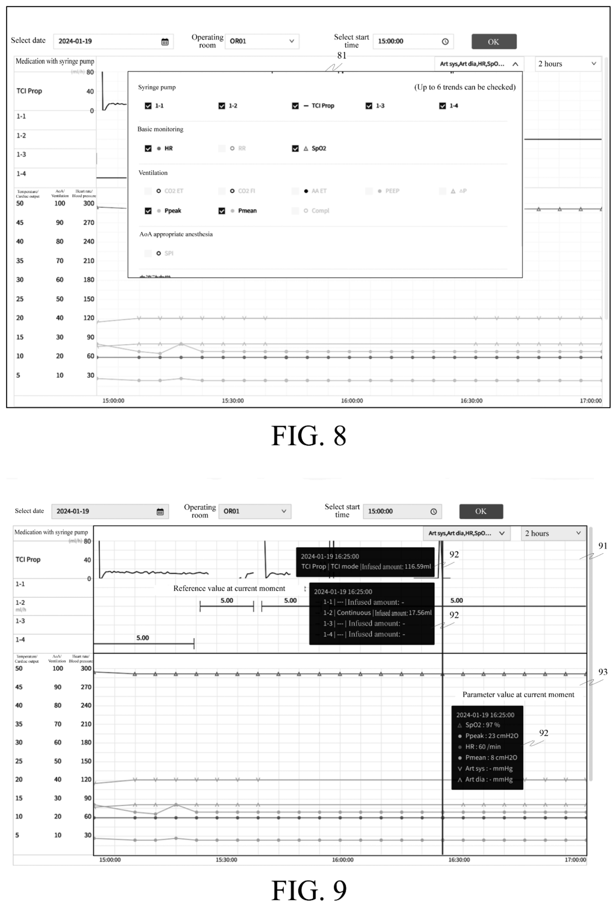

Refer to FIG. 8, which shows a schematic diagram of an interface of a first selection component provided by an exemplary embodiment of the present disclosure. The system displays a first selection component 81 on a current interface in an overlaid manner in response to the first selection component 81 being triggered. A plurality of first candidate physiological parameters are displayed in the first selection component 81. The plurality of first candidate physiological parameters include: 1. Medication with syringe pump: Rate variations of different syringe pumps, such as 1-1, 1-2, 1-3, and 1-4, as well as target-controlled infusion (TCI), in the unit of ml/h. 2. Basic monitoring: Heart rate (HR), respiratory rate (RR), and peripheral capillary oxygen saturation (SpO2). 3. Ventilation: A variety of respiration-related parameters, including: carbon dioxide end-tidal partial pressure (CO2 ET), anesthetic agent end-tidal (AA ET), positive end-expiratory pressure (PEEP), peak pressure (Ppeak), mean airway pressure (Pmean), and airway pressure ($\triangle$P). 4. AoA appropriate anesthesia: Parameters related to an anesthesia depth, such as a sedation probability index (SPI). In the current interface, the user may select the physiological parameter that the user wants to monitor by checking or unchecking. Once selection is completed, the system may use, based on user selection, one or more first candidate physiological parameters in the selected state as the first set of physiological parameters and display the corresponding trend chart.

Moreover, the trend chart provided by the embodiments of the present disclosure further has a function of triggering linked display. If the user selects any position in the trend chart, the system automatically positions and triggers linked display of other display positions (such as the waveform graph and the parameter list). That is, the system may display the waveform graph and/or the parameter list corresponding to a third moment (that is, a time point corresponding to the selected position in the trend chart) in response to any position in the trend chart being selected. In addition, in response to any position in the trend chart being selected, the system displays in the form of a pop-up box information of the first set of physiological parameters corresponding to the third moment.

Refer to FIG. 9, which shows a schematic diagram of an interface for trend chart-triggered linkage provided by an exemplary embodiment of the present disclosure. In a trend chart 91, the user may select a position of any data point. In response to any position in the trend chart 91 being selected, the system displays in the form of a pop-up box 92 information of a first set of physiological parameters corresponding to a time point of the position, and displays in a linked manner a waveform graph 93 and a parameter list (not shown in the figure) corresponding to the time point.

Features of the waveform graph include, but are not limited to: 1. The user can arbitrarily combine physiological parameters presented in the waveform graph according to own needs by means of the selection component. For a specific medical monitoring need, such flexibility allows the user to select the most relevant parameters for observation and analysis. 2. The system may respond to various user operations, including selecting a start moment, clicking a medical event, or clicking a data point on the trend chart, and display the corresponding waveform graph using a time point corresponding to the operation as the time center of the waveform graph. 3. The user can adjust a displayed time range of the waveform graph by sliding a slider or clicking a button. The slider allows the user to smoothly slide the slider to adjust the time range, and the button provides a small-scale adjustment manner. For example, each click of the button can move a display duration of the waveform graph by a time interval of 1 second, so that the user can accurately position a time period of interest. 4. The system adopts a segment loading solution in which physiological parameter data is divided into a plurality of data segments in chronological order. This design enables the system to intelligently load data as needed based on a user interaction manner, thereby improving data loading efficiency and user experience.

In some embodiments, if the user triggers a second selection component, the system, in response to the second selection component being triggered, displays the second selection component on a current interface in an overlaid manner. A plurality of second candidate physiological parameters are displayed in the second selection component. States of the second candidate physiological parameter include a selected state or an unselected state. After parameter selection is completed, one or more second candidate physiological parameters in the selected state are used as a second set of physiological parameters, and the corresponding waveform graph is displayed. It should be noted that a manner for triggering the second selection component may refer to the above manner for triggering the first selection component, and details are not described herein again.

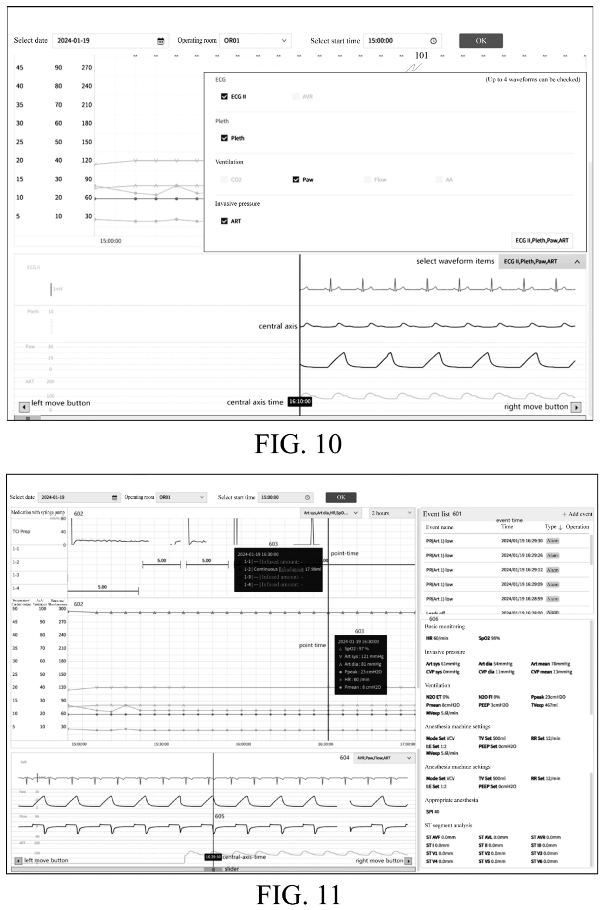

Refer to FIG. 10, which shows a schematic diagram of an interface of a second selection component provided by an exemplary embodiment of the present disclosure. The system displays a second selection component 101 on a current interface in an overlaid manner in response to the second selection component 101 being triggered. A plurality of second candidate physiological parameters are displayed in the second selection component 101. The plurality of second candidate physiological parameters include: 1. Electrocardiogram (ECG): Include electrocardiogram II (Electrocardiogram II, ECG II) and augmented vector right (Augmented Vector Right, AVR). 2. Plethysmography (Pleth): Include Pleth. 3. Ventilation: Include carbon dioxide ($CO_2$) in exhaled air, airway pressure (Paw), flow (English: flow), and anesthesia air (AA). 4. Invasive pressure: Arterial pressure trace (ART). In the current interface, the user may select the physiological parameter that the user wants to monitor by checking or unchecking. Once selection is completed, the system may use, based on user selection, one or more second candidate physiological parameters in the selected state as a second set of physiological parameters and display the corresponding waveform graph.

In some embodiments, the system may respond to various user operations, and display the corresponding waveform graph using a time point corresponding to the operation as the time center of the waveform graph, including but not limited to the following several possible implementations: the system, in response to a start moment being selected, displays the corresponding waveform graph using the start moment as the time center of the waveform graph, wherein the time center represents a midpoint of a displayed time range of the waveform graph; or, in response to any medical event among the medical events being selected, displays the corresponding waveform graph using a start moment of the selected medical event as the time center of the waveform graph; or, in response to any position in the trend chart being selected, displays the corresponding waveform graph using a moment corresponding to the selected position as the time center of the waveform graph. That is, the system allows the user to set the time center of the waveform graph by selecting the start moment, the medical event, and the specific position in the trend chart. The time center is the midpoint of the displayed time range of the waveform graph, ensuring that the user can view physiological parameter variations around the key time point. The system can respond to various user operations, including selecting the start moment, clicking the medical event, or clicking the data point on the trend chart. These operations trigger the system to update and display the waveform graph using the corresponding time point as the time center of the waveform graph. Once the time center is set, the system presents waveform data of each preset time period (such as 15 minutes) before and after the time point. This presentation manner provides the user with a clear snapshot of physiological parameter variations, so that the user can quickly grasp physiological states before and after the key time point.

Moreover, the waveform graph provided by the embodiments of the present disclosure further has a function of triggering linked display. If the user triggers a target control (such as an operation of clicking the button or sliding the slider) on the waveform graph, the system displays an adjusted waveform graph in response to the target control on the waveform graph being triggered, and display in a linked manner the parameter list corresponding to a fourth moment. The target control is configured to adjust a displayed time range of the waveform graph, and the fourth moment is the time center of the adjusted waveform graph.

Refer to FIG. 11, which shows a schematic diagram of an interface of waveform graph-triggered linkage provided by an exemplary embodiment of the present disclosure.

The system concurrently displays an event list 601, a trend chart 602, a waveform graph 604, and a parameter list 606 on a user interface, and displays in the form of a pop-up box 603 parameter values of a first set of physiological parameters in the trend chart 602. On the waveform graph 604, the user may adjust a central axis time 605 of the waveform graph 604 by clicking a button, allowing the user to make fine adjustments on a timeline. The button may include a left button and a right button. The left button is designed to move the central axis time 605 of the waveform graph 604 forward by a preset time interval (for example, -1 second per operation). Corresponding to the left button, the right button is configured to move the central axis time 605 of the waveform graph 604 backward by the same preset time interval. The user may alternatively adjust the central axis time 605 of the waveform graph 604 by sliding a slider. The slider provides a quick adjustment manner that allows the user to quickly slide along the timeline. When the user adjusts the central axis time 605 of the waveform graph 604 to 16:29:30 by clicking the button or sliding the slider, the system recognizes this operation. In response to adjusting the central axis time 605, the system automatically updates the parameter list 606 to present parameter values of a third set of physiological parameters corresponding to the adjusted central axis time 605.

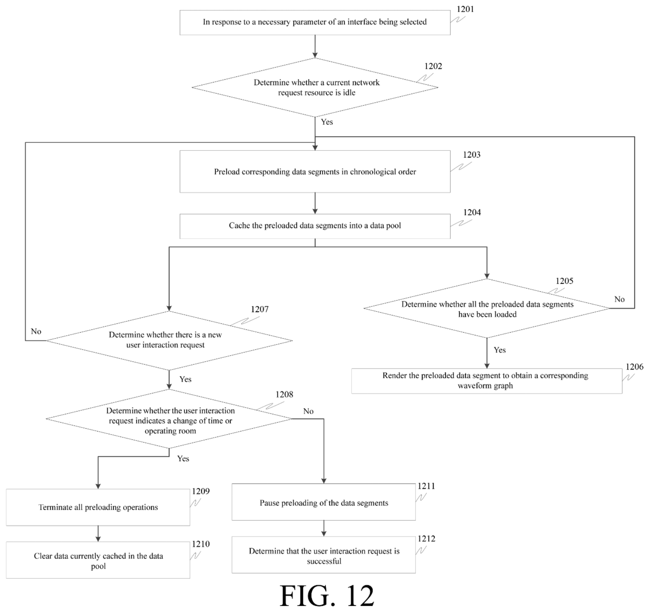

A data preloading process of the waveform graph is further described below. Refer to FIG. 12, which shows a flowchart of a data preloading process of a waveform graph provided by an exemplary embodiment of the present disclosure. This embodiment is illustrated by taking the method being used in the above data playback system as an example. The method includes the following steps:

Step 1201: Respond to a necessary parameter on an interface being selected.

The user selects the necessary parameter on the interface, including but not limited to selecting a date, an operating room, a start time, and the like. These parameters are prerequisites for data loading and waveform graph display. The system responds to the necessary parameter on the interface being selected.

Step 1202: Determine whether a current network request resource is idle.

Before data loading, the system needs to check whether the network request resource is idle, to ensure that a data loading process is not hindered due to resource conflicts. If the current network request resource is idle, step 1203 is executed. Otherwise, the process is terminated.

Step 1203: Preload corresponding data segments in chronological order.

The system divides physiological parameter data into a plurality of consecutive data segments in chronological order. When the user selects a specific time interval, that is, a first time period, the system responds to the selection and sequentially preloads data segments corresponding to the first time period in chronological order.

Step 1204: Cache the preloaded data segments into a data pool.

The system caches the preloaded data segments into the data pool in the form of key-value pairs (English: key-value). The form of key-value pairs is a data storage manner, wherein each data item is composed of a unique key (English: key) and a value (English: value). In the embodiment of the present disclosure, the key may represent a unique identifier (such as a timestamp or a segment number) of the data segment, and the value is the actual data segment.

Step 1205: Determine whether all the preloaded data segments have been loaded.

Step 1206: If all the preloaded data segments have been loaded, render the preloaded data segments to obtain the corresponding waveform graph.

If all the preloaded data segments are not completely loaded, step 1203 continues to be executed, to preload remaining data segments in chronological order.

Step 1207: Determine whether there is a new user interaction request.

The system detects whether there is the new user interaction request. If the new user interaction request exists, step 1208 is executed. Otherwise, step 1203 continues to be executed.

Step 1208: If the new user interaction request exists, determine whether the user interaction request indicates a change of time or operating room.

In some embodiments, the system may pause preloading of the data segments in response to the user interaction request; and/or in response to the first time period being changed, data currently cached in the data pool is cleared. That is, if the user performs a new interaction operation, the system may further determine whether the user interaction request involves changing the time or the operating room. If the user interaction request indicates a change of time or operating room, step 1209 is executed. Otherwise, step 1211 is executed.

Step 1209: If the user interaction request indicates a change of time or operating room, terminate all preloading operations.

If the user interaction request indicates a change of time or the operating room, the system terminates all current preloading operations to prepare for loading a new data segment.

Step 1210: Clear the data currently cached in the data pool.

In response to the user interaction request, the system clears the data currently cached in the data pool, and re-executes step 1203 to start preloading the new data segment.

Step 1211: If the user interaction request does not indicate a change of time or operating room, pause preloading of the data segments.

If the user interaction request does not indicate a change of time or operating room, the system pauses preloading of the current data segments.

Step 1212: Determine that the user interaction request is successful.

After it is determined that the user interaction request is successful, and the user completes an interaction operation, the system may continue to preload the data segments.

The system can intelligently adjust priorities of the data preloading request and the user interaction operation by implementing a task scheduling mechanism. This dynamic adjustment ensures page running fluency, and simultaneously optimizes the user's interactive experience, so that the user experiences a more rapid and seamless service during use.

In some embodiments, a segment loading solution is used in the data preloading process of the waveform graph, that is, the physiological parameter data is divided into a plurality of data segments, and corresponding data is preloaded and cached as needed according to the interaction operation of the user, and ultimately rendered to obtain a required waveform graph.

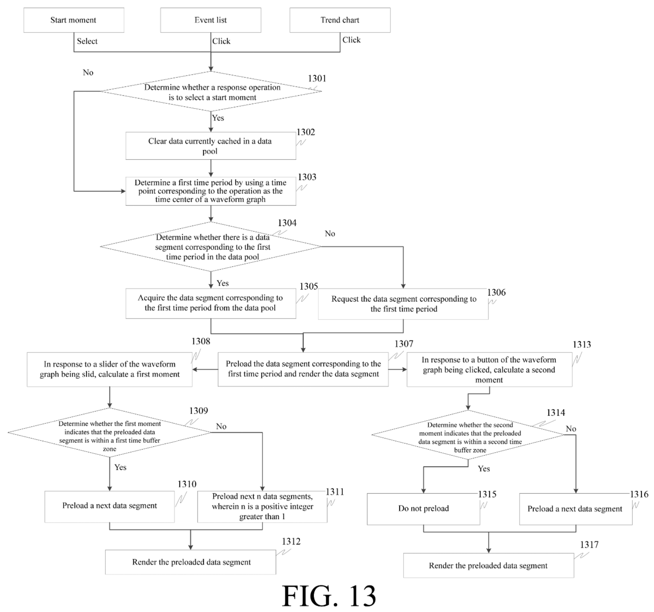

The segment loading solution of the waveform graph is further described below. Refer to FIG. 13, which shows a flowchart of a segment loading solution of a waveform graph provided by an exemplary embodiment of the present disclosure. This embodiment is illustrated by taking the method being use in the above data playback system as an example. The method includes the following steps:

Step 1301: Determine whether a response operation is to select a start moment.

The system can respond to various user operations, including selecting a start moment, clicking a medical event, or clicking a data point on the trend chart. These operations trigger the system to update and display the waveform graph using the corresponding time point as the time center of the waveform graph. Therefore, the system first determines whether the response operation is to select the start moment. If yes, step 1302 is executed; and otherwise, step 1303 is executed.

Step 1302: Clear data currently cached in a data pool.

If the user selects the start moment, the system clears the data currently cached in the data pool.

Step 1303: Determine a first time period using a time point corresponding to the operation as the time center of the waveform graph.

Regardless of whether the user operation is to select the start moment, click the medical event, or click the data point on the trend chart, the system uses the time point corresponding to the operation as the time center of the waveform graph, and based on the time center, the system determines a first time period. The first time period usually includes preset time periods before and after the time center. For example, if the preset time period is 15 minutes, the first time period includes data from 15 minutes before the time center to 15 minutes after the time center.

Step 1304: Determine whether there is a data segment corresponding to the first time period in the data pool.

The system determines whether there is the data segment corresponding to the first time period in the data pool. If yes, the system directly acquires data from the data pool, that is, step 1305 is executed. If no, the system requests a new data segment, that is, step 1306 is executed.

Step 1305: Acquire the data segment corresponding to the first time period from the data pool.

Step 1306: Request the data segment corresponding to the first time period.

Step 1307: Preload the data segment corresponding to the first time period and render the data segment.

The data segment corresponding to the first time period is preloaded, the preloaded data segment is cached into the data pool, and the preloaded data segment is rendered to obtain a waveform graph of the first time period.

Step 1308: In response to a slider of the waveform graph being slid, calculate a first moment.

When the user slides the slider on the waveform graph, the system calculates the first moment, and the moment becomes the time center of the adjusted waveform graph. Operating the slider allows the user to intuitively control a timeline of the waveform graph to view physiological parameter variations at a specific time point.

Step 1309: Determine whether the first moment indicates that the preloaded data segment is within a first time buffer zone.

The system then determines whether the calculated first moment indicates that the preloaded data segment is within the first time buffer zone. Definition of the first time buffer zone may be understood as a time range determined based on the current time center (the first moment) and a total displayed duration of the waveform graph in display of the waveform graph. A purpose of the buffer zone is to determine whether the preloaded data segment is sufficient to cover a time range that the waveform graph needs to display after the slider is slid, thereby determining whether more data needs to be loaded.

Optionally, a condition for determining whether the first moment indicates that the preloaded data segment is within the first time buffer zone may be set as follows:

$$t_s - T_{n1} - \frac{L_t}{2} \geq 0$$

wherein $t_s$ is an end moment of the last preloaded data segment, $T_{n1}$ is the first moment, and $L_t$ is the total displayed duration of the waveform graph. Taking this relation as an example, it can be considered that a boundary of the first time buffer zone is $T_{n1} + \frac{L_t}{2}$. When the above determination condition is satisfied, it means that the first moment indicates that the preloaded data segment is within the first time buffer zone. Then, step 1310 is executed. Otherwise, step 1311 is executed.

It should be noted that the above determination condition is only used as an example condition, and different determination conditions may be set according to actual needs, which is not limited in the embodiments of the present disclosure.

Step 1310: If the first moment indicates that the preloaded data segment is within the first time buffer zone, preload a next data segment.

If the first moment indicates that the preloaded data segment is within the first time buffer zone, it means that the preloaded data segment is sufficient to cover the time range that the waveform graph needs to display. In this case, the system may preload the next data segment. Reasons for this include: 1. By preloading the next data segment, the system can prepare data of the next time range that the user may slide to in advance, thereby reducing time for the user to wait for data loading; 2. The currently preloaded data segment is already within the first time buffer zone, so there is no need to load too many new data segments, which can prevent unnecessary data loading and improve a response speed of a user interface.

Step 1311: If the first moment indicates that the preloaded data segment is not within the first time buffer zone, preload next n data segments, wherein n is a positive integer greater than 1.

If the first moment indicates that the preloaded data segment is not within the first time buffer zone, it means that the user has slid to a new region that is not covered by the currently preloaded data. In this case, the system is going to preload the next n data segments. Reasons for this include: 1. Adapt to user behavior: The user may have performed a large sliding operation, and data within a farther time range needs to be viewed. Preloading a plurality of data segments may ensure that data can be quickly accessed even the user continues to slide. 2. Reduce waiting time: By preloading a plurality of data segments at one time, a number of times that the user encounters data loading delays during a sliding process can be reduced, improving user satisfaction.

Step 1312: Render the preloaded data segments.

Regardless of whether a single data segment or a plurality of data segments are preloaded, the system caches a preloaded data segment into the data pool and renders the preloaded data segment. This step ensures real-time updates and display of the waveform graph, providing the user with a continuous and detailed view of physiological parameter variations.

Step 1313: In response to a button on the waveform graph being clicked, calculate a second moment.

When the user clicks a button on the waveform graph, the system calculates a second moment, and the moment becomes the time center of the adjusted waveform graph. The button allows the user to quickly move a displayed time range of the waveform graph at fixed time intervals.

Step 1314: Determine whether the second moment indicates that the preloaded data segment is within a second time buffer zone.

The system then determines whether the calculated second moment indicates that the preloaded data segment is within the second time buffer zone. Similar to the slider operation, definition of the second time buffer zone may be understood as a time range determined based on the current time center (the second moment) and the total displayed duration of the waveform graph in display of the waveform graph. A purpose of the buffer zone is to determine whether the preloaded data segment is sufficient to cover a time range that the waveform graph needs to display after the button is clicked, thereby determining whether more data needs to be loaded.

Optionally, a condition for determining whether the second moment indicates that the preloaded data segment is within the second time buffer zone may be set as follows:

$$t_s - T_{n2} - L_t \geq 0$$

wherein $t_s$ is an end moment of the last preloaded data segment, $T_{n2}$ is the second moment, and $L_t$ is the total displayed duration of the waveform graph. Taking this relation as an example, it can be considered that a boundary of the second time buffer zone is $T_{n2}+L_t$. When the above determination condition is satisfied, it means that the second moment indicates that the preloaded data segment is within the second time buffer zone. Then, step 1315 is executed. Otherwise step 1316 is executed.

It should be noted that the above determination condition is only used as an example condition, and different determination conditions may be set according to actual needs, which is not limited in the embodiments of the present disclosure.

It should be further noted that in a waveform graph operation of the system, the slider and the button provide two different user interaction manners, which are respectively adapted to different usage scenarios and user needs. The slider allows users to navigate quickly and continuously over time. When the users use the slider, they may slide with a large amplitude to quickly position a specific region on the waveform graph. To accommodate this rapid movement, the system may employ a more aggressive preloading strategy. That is, the system preloads more data segments when the slider moves, ensuring that data of a new region is ready even if the user slides the slider rapidly, thereby reducing waiting time and providing smoother user experience, so that the user hardly feel any delay during a sliding process. The button is used for more precise or controlled time jumps. When clicking the button, the user is usually looking for a specific time point or making a small movement. Therefore, the system may employ a more conservative preloading strategy for a button operation. That is, when the user clicks the button, the system calculates the second moment and determines whether new data needs to be preloaded. If the second moment indicates that the preloaded data segment is within the second time buffer zone, the system may execute step 1315, that is, no additional preloading is performed, because the current data is sufficient for the user's viewing needs. If the second moment indicates that the preloaded data segment is not within the second time buffer zone, the system may execute step 1316, that is, the next data segment is preloaded. This strategy prevents unnecessary data loading, improving system efficiency while ensuring that the user can immediately acquire required data after clicking the button.

By means of this differentiated preloading strategy, the system can flexibly respond to different user operations, which not only meets requirements for data continuity during fast sliding, but also ensures efficiency and response speed during precise jumps. This design considers user operation diversity, and aims to provide more personalized and optimized user experience.

Step 1315: If the second moment indicates that the preloaded data segment is within the second time buffer zone, skip preloading.

If the second moment indicates that the preloaded data segment is within the second time buffer zone, it means that the currently displayed data is sufficient to cover a range that the user may view. In this case, the system may not perform additional preloading.

Step 1316: If the second moment indicates that the preloaded data segment is not within the second time buffer zone, preload a next data segment.

If the second moment indicates that the preloaded data segment is not within the second time buffer zone, it means that the user has moved to a new region that is not covered by the currently preloaded data. In this case, the system is going to preload the next data segment.

Step 1317: Render the preloaded data segment.

The system caches the preloaded data segment into the data pool and renders the preloaded data segment. This step ensures real-time updates and display of the waveform graph, providing the user with a continuous and detailed view of physiological parameter variations.

In summary, the data playback method provided by the embodiments of the present disclosure implements efficient and intuitive medical data presentation by means of the following strategies:

1. Data visualization: The medical data collected by the plurality of medical devices are presented on the same screen in the form of the event list, the trend chart, the waveform graph, and the parameter list, which enhances data intuitiveness and readability.

2. Automatic association and rapid positioning: All functions are automatically associated, so that data can be quickly positioned. For example, when any medical event is clicked, the system automatically positions the physiological parameter data of the plurality of medical devices at the same moment based on the occurrence time of the medical event. The linkage sequence starts from the event list, to the trend chart, the waveform graph, and the parameter list.

3. Free collocation of parameter data: Considering a huge amount of data generated by the plurality of medical devices, the system automatically filters parameters that have data of the plurality of medical devices within the time period selected by the user, and provides a function of selecting and freely collocating parameter data of the different medical devices.

4. Algorithm and preloading concept: Combining an algorithm and a preloading concept significantly shortens time for data loading, presentation, and interaction, improving user experience.

Embodiments of the present disclosure further provide a data playback system. The system includes:

a processor;

a display, configured to receive an instruction from the processor to perform display; and

a memory, configured to store instructions executable by the processor, wherein

the processor is configured to implement the following operations when executing the instructions stored in the memory:

acquiring medical data collected by a plurality of medical devices, wherein the medical data includes physiological parameter data of a target subject within a period of time and a medical event related to the target subject;

preprocessing the physiological parameter data, wherein the preprocessing includes:

dividing the physiological parameter data into a plurality of data segments in chronological order;

in response to the selection of a first time period, preloading a data segment corresponding to the first time period, and caching the preloaded data segment into a data pool; and

rendering the preloaded data segment to obtain a waveform graph of the first time period; and

concurrently displaying the waveform graph of the first time period and the medical event.

In a possible implementation, the processor is further configured to:

in response to a user interaction request, pause preloading of the data segment; and/or,

and/or in response to the first time period being changed, clear data currently cached in the data pool.

In another possible implementation, the processor is further configured to:

in response to a slider of the waveform graph being slid, calculate a first moment, wherein the slider is configured to slidably adjust a displayed time range of the waveform graph, and the first moment is the time center of the adjusted waveform graph;

if the first moment indicates that the preloaded data segment is within a first time buffer zone, preload a next data segment; and

if the first moment indicates that the preloaded data segment is not within the first time buffer zone, preload next n data segments, wherein n is a positive integer greater than 1.

In another possible implementation, the processor is further configured to:

in response to a button on the waveform graph being clicked, calculate a second moment, wherein the button is configured to adjust a displayed time range of the waveform graph at fixed time intervals, and the second moment is the time center of the adjusted waveform graph;

if the second moment indicates that the preloaded data segment is within a second time buffer zone, skip preloading; and

if the second moment indicates that the preloaded data segment is not within the second time buffer zone, preload a next data segment.

In another possible implementation, the processor is further configured to:

in response to a time period to be played back being selected, concurrently display the medical events within the selected time period; and

in response to any medical event among the medical events being selected, display in a linked manner data of the plurality of medical devices corresponding to the selected medical event.

In another possible implementation, the physiological parameter data includes a plurality of integrated parameters, and a display form of the plurality of integrated parameters further includes at least one of a trend chart and a parameter list, the trend chart being configured to display a trend of a first set of physiological parameters of the target subject varying over time within a second time period, the waveform graph being configured to display a waveform of a second set of physiological parameters of the target subject varying over time within the first time period, the parameter list being configured to display a third set of physiological parameters of the target subject at a target moment, the first time period being contained in the second time period, and the target moment being a time point in the second time period.

In another possible implementation, the processor is further configured to:
in response to the second time period being changed, filtering a plurality of first candidate physiological parameters that have data within the changed second time period, wherein the plurality of first candidate physiological parameters are used as a selection range for customizing the first set of physiological parameters.

In another possible implementation, the processor is further configured to:

in response to a first selection component being triggered, display the first selection component on a current interface in an overlaid manner, wherein a plurality of first candidate physiological parameters are displayed in the first selection component, and states of the first candidate physiological parameter includes a selected state or an unselected state; and

after parameter selection is completed, use one or more first candidate physiological parameters in the selected state as the first set of physiological parameters, and display the corresponding trend chart.

In another possible implementation, the processor is further configured to:
in response to any position in the trend chart being selected, display in a linked manner the waveform graph and/or the parameter list corresponding to a third moment, wherein the third moment is a time point corresponding to the selected position in the trend chart.

In another possible implementation, the processor is further configured to:
in response to any position in the trend chart being selected, display in the form of a pop-up box information of the first set of physiological parameters corresponding to the third moment.

In another possible implementation, the processor is further configured to:

in response to a second selection component being triggered, display the second selection component on a current interface in an overlaid manner, wherein a plurality of second candidate physiological parameters are displayed in the second selection component, and states of the second candidate physiological parameters include a selected state or an unselected state; and

after parameter selection is completed, use one or more second candidate physiological parameters in the selected state as the second set of physiological parameters, and display the corresponding waveform graph.

In another possible implementation, the processor is further configured to:

in response to a start moment being selected, display the corresponding waveform graph using the start moment as the time center of the waveform graph, wherein the time center represents a midpoint of a displayed time range of the waveform graph; or,

in response to any medical event among the medical events being selected, display the corresponding waveform graph using a start moment of the selected medical event as the time center of the waveform graph; or,

in response to any position in the trend chart being selected, display the corresponding waveform graph using a moment corresponding to the selected position as the time center of the waveform graph.

In another possible implementation, the processor is further configured to:
in response to a target control on the waveform graph being triggered, display the adjusted waveform graph, and display in a linked manner the parameter list corresponding to a fourth moment.

The target control is configured to adjust a displayed time range of the waveform graph, and the fourth moment is the time center of the adjusted waveform graph.

In another possible implementation, the plurality of medical devices include a plurality of devices selected from a group including a syringe pump, a monitor, an electrocardiograph, an electroencephalograph, a ventilator, and an anesthesia machine.

In another possible implementation, the data playback system is a system independent of the plurality of medical devices. The data playback system performs data exchange with the plurality of medical devices by means of a server. The server is configured to integrate and process data collected by the plurality of medical devices.

It should be noted that specific operation manners of the data playback system have been described in detail in the relevant method embodiments, and will not be described in detail herein. In practical applications, a content structure of the data playback system may be divided into different functional modules according to actual needs to complete all or part of the functions described above.

Embodiments of the present disclosure further provide a non-volatile computer-readable storage medium, having computer program instructions stored thereon, wherein the computer program instructions, when executed by a processor, implement the method provided in the above embodiments.

Embodiments of the present disclosure further provide a computer program product, including computer-readable codes or a non-volatile computer-readable storage medium loaded with computer-readable codes, wherein when the computer-readable codes are run in a processor of a computing device, the processor in the computing device executes the method provided in the above embodiments.

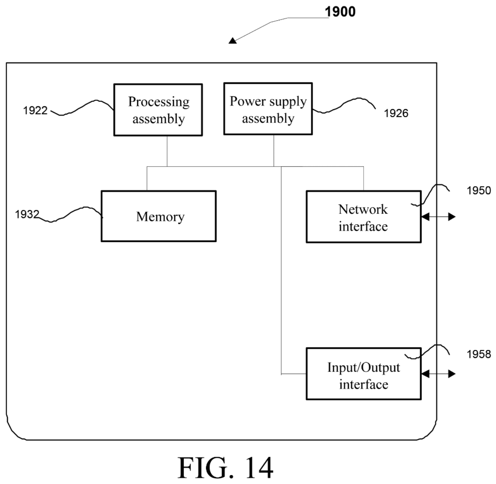

FIG. 14 is a block diagram of an apparatus 1900 according to an exemplary embodiment. For example, the apparatus 1900 is configured to execute the method described in the above method embodiments, and may be provided as a server or a terminal device. Referring to FIG. 14, the apparatus 1900 includes a processing assembly 1922 that further includes one or more processors, and a memory resource represented by a memory 1932 for storing instructions executable by the processing assembly 1922, such as an application. The application stored in the memory 1932 can include one or more modules each corresponding to a set of instructions. In addition, the processing assembly 1922 is configured to execute instructions to execute the above method.

The apparatus 1900 may further include a power supply assembly 1926 configured to execute power management of the apparatus 1900, a wired or wireless network interface 1950 configured to connect the apparatus 1900 to a network, and an input/output interface (I/O interface) 1958. The apparatus 1900 may operate based on an operating system stored in the memory 1932, for example, Windows Server™, Mac OS X™, Unix™, Linux™, or FreeBSD™.

In an exemplary embodiment, a non-volatile computer-readable storage medium is further provided, for example, the memory 1932 including computer program instructions that are executable by the processing assembly 1922 of the apparatus 1900 to complete the above method.

The present disclosure may be a system, a method and/or a computer program product. The computer program product may include a computer-readable storage medium, having computer-readable program instructions thereon for causing a processor to implement various aspects of the present disclosure.

The computer-readable storage medium can be a tangible device that can hold and store instructions used by an instruction execution device. The computer-readable storage medium may be, for example, but is not limited to an electrical storage device, a magnetic storage device, an optical storage device, an electromagnetic storage device, a semiconductor memory device, or any suitable combination thereof. More specific examples (a non-exhaustive list) of the computer-readable storage medium include: a portable computer disk, a hard disk, a random access memory (RAM), a read only memory (ROM), a erasable programmable read only memory (EPROM or flash memory), a static random access memory (SRAM), a portable compact disc read-only memory (CD ROM), a digital versatile disk (DVD), a memory stick, a floppy disk, mechanical coding equipment, such as a punch card having instructions stored thereon or a structure of bumps within recessions, and any suitable combination thereof. The computer-readable storage medium used herein is not interpreted as transient signals themselves, such as radio waves or other freely propagated electromagnetic waves, electromagnetic waves propagated through a waveguide or other transmission media (e.g., light pulses passing through a fiber optic cable), or electrical signals transmitted through electric wires.

The computer-readable program instructions described herein may be downloaded from a computer-readable storage medium to various computing/processing devices or downloaded to an external computer or external storage device via a network such as the Internet, a local area network, a wide area network and/or a wireless network. The network may include copper transmission cables, fiber transmission, wireless transmission, routers, firewalls, switches, gateway computers, and/or edge servers. A network adapter card or a network interface in each computing/processing device receives computer-readable program instructions from the network and forwards the computer-readable program instructions, for storing them in a computer-readable storage medium in each computing/processing device.

Computer program instructions for executing the operations of the present disclosure can be assembly instructions, instruction set architecture (ISA) instructions, machine instructions, machine related instructions, microcode, firmware instructions, state setting data, or source code or object code written in any combination of one or more programming languages, the programming language including object oriented programming languages such as Smalltalk, C++ and the like, and conventional procedural programming languages such as the "C" language or similar programming languages. The computer-readable program instructions can be executed entirely or partly on a user computer, executed as a stand-alone software package, executed partly on a user computer and partly on a remote computer, or executed entirely on a remote computer or server. In the case of a remote computer, the remote computer may be connected to the user's computer through any kind of network, including a local area network (LAN) or a wide area network (WAN). Alternatively, it can be connected to an external computer (for example, using an Internet service provider to connect via the Internet). In some embodiments, an electronic circuit, for example, a programmable logic circuit, a field-programmable gate array (FPGA), or a programmable logic array (PLA), may execute the computer-readable program instructions by utilizing state information of the computer-readable program instructions to personalize the electronic circuit, in order to implement various aspects of the present disclosure.

The aspects of the present disclosure are described herein with reference to the flowcharts and/or block diagrams of the methods, apparatuses (systems), and computer program products according to the embodiments of the present disclosure. It should be understood that each block of the flowcharts and/or block diagrams and combinations of various blocks in the flowcharts and/or block diagrams can be implemented by computer-readable program instructions.

These computer-readable program instructions may be provided to a processor of a general-purpose computer, a special-purpose computer, or other programmable data processing apparatuses, to produce a machine, so that these instructions, when executed by the processor of the computer or other programmable data processing apparatuses, produce an apparatus for implementing the functions/actions specified in one or more blocks of the flowcharts and/or block diagrams. Also, these computer-readable program instructions may be stored in a computer-readable storage medium. These instructions cause a computer, a programmable data processing apparatus, and/or other devices to work in a specific manner. Thus, the computer-readable medium storing the instructions includes an artifact, including instructions that implement various aspects of the functions/actions specified in one or more the flowcharts and/or block diagrams.

The computer-readable program instructions may also be loaded onto a computer, other programmable data processing apparatuses, or other devices, such that the computer, other programmable data processing apparatuses or other devices execute a series of operational steps, to generate a computer-implemented process, such that the functions/actions specified in one or more of the flowcharts and/or block diagrams are implemented by the instructions executed on the computer, other programmable data processing apparatuses, or other devices.

The flowcharts and block diagrams in the accompanying drawings illustrate system architectures, functions, and operations of possible implementations of the system, method, and computer program product according to a plurality of embodiments of the present disclosure. In this regard, each block in the flowcharts or block diagrams may represent a portion of a module, program segment, or instruction that contains one or more executable instructions for implementing the specified logical functions. In some alternative implementations, the functions denoted in the blocks can also occur in a different order than that illustrated in the drawings. For example, two consecutive blocks can actually be executed substantially in parallel, and sometimes can also be executed in a reverse order, depending upon the functions involved. It should also be noted that each block of the block diagrams and/or flowcharts, and combinations of blocks in the block diagrams and/or flowcharts can be implemented in a dedicated hardware-based system that executes the specified function or action, or can be implemented by a combination of dedicated hardware and computer instructions.

The embodiments of the present disclosure have been described above. The foregoing description is illustrative rather than limiting, and is not limited to the disclosed embodiments. Many modifications and variations are apparent to those of ordinary skill in the art without departing from the scope and spirit of the embodiments illustrated. The selection of terms used herein is intended to best explain the principles, practical applications, or improvements to the technologies in the market of the embodiments, or to enable others of ordinary skill in the art to understand the embodiments disclosed herein.

**Claims**

1. A data playback method, **characterized by** comprising:

   acquiring medical data collected by a plurality of medical devices, wherein the medical data comprises physiological parameter data of a target subject within a period of time and a medical event related to the target subject;
   preprocessing the physiological parameter data, wherein the preprocessing comprises:

   dividing the physiological parameter data into a plurality of data segments in chronological order;
   in response to the selection a first time period, preloading a data segment corresponding to the first time period, and caching the preloaded data segment into a data pool; and
   rendering the preloaded data segment to obtain a waveform graph of the first time period; and

   concurrently displaying the waveform graph of the first time period and the medical event.

2. The method according to claim 1, further comprising:

   in response to a user interaction request, pausing preloading of the data segment; and/or,
   in response to the first time period being changed, clearing data currently cached in the data pool.

3. The method according to claim 1, further comprising:

   in response to a slider of the waveform graph being slid, calculating a first moment, wherein the slider is configured to slidably adjust a displayed time range of the waveform graph, and the first moment is the time center of the adjusted waveform graph;
if the first moment indicates that the preloaded data segment is within a first time buffer zone, preloading a next data segment; and
if the first moment indicates that the preloaded data segment is not within the first time buffer zone, preloading next n data segments, wherein n is a positive integer greater than 1.

4. The method according to claim 1, further comprising:

   in response to a button on the waveform graph being clicked, calculating a second moment, wherein the button is configured to adjust a displayed time range of the waveform graph at fixed time intervals, and the second moment is the time center of the adjusted waveform graph;
   if the second moment indicates that the preloaded data segment is within a second time buffer zone, skipping preloading; and
   if the second moment indicates that the preloaded data segment is not within the second time buffer zone, preloading a next data segment.

5. The method according to claim 1, further comprising:

   in response to a time period to be played back being selected, concurrently displaying the medical events within the selected time period; and
   in response to any medical event among the medical events being selected, displaying in a linked manner data of the plurality of medical devices corresponding to the selected medical event.

6. The method according to claim 1, wherein the physiological parameter data comprises a plurality of integrated parameters, and a display form of the plurality of integrated parameters further comprises at least one of a trend chart and a parameter list, the trend chart being configured to display a trend of a first set of physiological parameters of the target subject varying over time within a second time period, the waveform graph being configured to display a waveform of a second set of physiological parameters of the target subject varying over time within the first time period, the parameter list being configured to display a third set of physiological parameters of the target subject at a target moment, the first time period being contained within the second time period, and the target moment being a time point within the second time period.

7. The method according to claim 6, further comprising:
   in response to the second time period being changed, filtering a plurality of first candidate physiological parameters that have data within the changed second time period, wherein the plurality of first candidate physiological parameters are used as a selection range for customizing the first set of physiological parameters.

8. The method according to claim 6, further comprising:

in response to a first selection component being triggered, displaying the first selection component on a current interface in an overlaid manner, wherein a plurality of first candidate physiological parameters are displayed in the first selection component, and states of a first candidate physiological parameter comprise a selected state or an unselected state; and after parameter selection is completed, using one or more of the first candidate physiological parameters in the selected state as the first set of physiological parameters, and displaying the corresponding trend chart.

9. The method according to claim 6, further comprising: in response to any position in the trend chart being selected, displaying in a linked manner the waveform graph and/or the parameter list corresponding to a third moment, wherein the third moment is a time point corresponding to the selected position in the trend chart.

10. The method according to claim 9, further comprising: in response to any position in the trend chart being selected, displaying in the form of a pop-up box information of the first set of physiological parameters corresponding to the third moment.

11. The method according to claim 6, further comprising:

in response to a second selection component being triggered, displaying the second selection component on a current interface in an overlaid manner, wherein a plurality of second candidate physiological parameters are displayed in the second selection component, and states of a second candidate physiological parameter comprise a selected state or an unselected state; and after parameter selection is completed, using one or more of the second candidate physiological parameters in the selected state as the second set of physiological parameters, and displaying the corresponding waveform graph.

12. The method according to claim 6, further comprising:

in response to a start moment being selected, displaying the corresponding waveform graph using the start moment as the time center of the waveform graph, wherein the time center represents a midpoint of a displayed time range of the waveform graph; or, in response to any medical event among the medical events being selected, displaying the corresponding waveform graph using a start moment of the selected medical event as the time center of the waveform graph; or, in response to any position in the trend chart being selected, displaying the corresponding waveform graph using a moment corresponding to the selected position as the time center of the waveform graph.

13. The method according to claim 6, further comprising:

in response to a target control on the waveform graph being triggered, displaying the adjusted waveform graph, and displaying in a linked manner the parameter list corresponding to a fourth moment; wherein the target control is configured to adjust a displayed time range of the waveform graph, and the fourth moment is the time center of the adjusted waveform graph.

14. A data playback system, **characterized by** comprising:

a processor; a display, configured to receive an instruction from the processor to perform display; and a memory, configured to store instructions executable by the processor, wherein the processor is configured to, when executing the instructions stored in the memory, implement the method according to any one of claims 1 to 13 .

15. A non-volatile computer-readable storage medium, having computer program instructions stored thereon, **characterized in that** the computer program instructions, when executed by a processor, implement the method according to any one of claims 1 to 13.

| A plurality of medical devices 12 | Server 14 | Data playback system 16 |

## FIG. 1

Acquire medical data collected by a plurality of medical devices, wherein the medical data includes physiological parameter data of a target subject within a period of time and a medical event related to the target subject — 201

Preprocess the physiological parameter data, wherein the preprocessing includes: divide the physiological parameter data into a plurality of data segments in chronological order; in response to the selection a first time period, preload a data segment corresponding to the first time period, and cache the preloaded data segment into a data pool; and render the preloaded data segment to obtain a waveform graph of the first time period — 202

Concurrently display the waveform graph of the first time period and the medical event — 203

## FIG. 2

Interface 30

| Trend chart 33 | Event list 31 |
| Waveform graph 32 | Parameter list 34 |

## FIG. 3

FIG. 4

FIG. 5

FIG. 6

FIG. 7

FIG. 8

FIG. 9

FIG. 10

FIG. 11

FIG. 12

| Start moment | Event list | Trend chart |
|---|---|---|

Select | Click | Click

No

**1301** Determine whether a response operation is to select a start moment

Yes

**1302** Clear data currently cached in a data pool

**1303** Determine a first time period by using a time point corresponding to the operation as the time center of a waveform graph

**1304** Determine whether there is a data segment corresponding to the first time period in the data pool — No

Yes

**1305** Acquire the data segment corresponding to the first time period from the data pool

**1306** Request the data segment corresponding to the first time period

**1307** Preload the data segment corresponding to the first time period and render the data segment

**1308** In response to a slider of the waveform graph being slid, calculate a first moment

**1313** In response to a button of the waveform graph being clicked, calculate a second moment

**1309** Determine whether the first moment indicates that the preloaded data segment is within a first time buffer zone — No

Yes

**1314** Determine whether the second moment indicates that the preloaded data segment is within a second time buffer zone — No

Yes

**1310** Preload a next data segment

**1311** Preload next n data segments, wherein n is a positive integer greater than 1

**1315** Do not preload

**1316** Preload a next data segment

**1312** Render the preloaded data segment

**1317** Render the preloaded data segment

## FIG. 13

**1900**

FIG. 14

Europäisches
Patentamt

European
Patent Office

Office européen
des brevets

# EUROPEAN SEARCH REPORT

**Application Number**

EP 25 22 1371

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2017/059596 A1 (SHENZHEN MINDRAY BIOMEDICAL ELECTRONICS CO LTD [CN]) 13 April 2017 (2017-04-13) * figures 1-3, 6 * * page 11 - page 12 * ----- | 1-15 | INV. G16H10/40 G16H10/60 G16H15/00 |
| X | US 2012/278099 A1 (KELLY LISA [US] ET AL) 1 November 2012 (2012-11-01) * figures 2-8, 16 * * paragraphs [0026] - [0034] * ----- | 1-15 | |
| X | US 2020/121199 A1 (FREEMAN GARY A [US] ET AL) 23 April 2020 (2020-04-23) * figures 1a-d, 5, 6a, b, 7a, b, 8, 9, 10a, 11, 12 * * paragraphs [0003] - [0010] * ----- | 1-15 | |

TECHNICAL FIELDS
SEARCHED        (IPC)

G16H

The present search report has been drawn up for all claims

| Place of search | Date of completion of the search | Examiner |
|---|---|---|
| Munich | 17 April 2026 | Trattner, Barbara |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.......................................................................................

& : member of the same patent family, corresponding
    document

EPO FORM 1503 03.82 (P04C01)

# EP 4 760 732 A1

## ANNEX TO THE EUROPEAN SEARCH REPORT
## ON EUROPEAN PATENT APPLICATION NO.

EP 25 22 1371

17-04-2026

| Patent document cited in search report | | Publication date | Patent family member(s) | | Publication date |
|---|---|---|---|---|---|
| WO 2017059596 | A1 | 13-04-2017 | CN | 107847176 A | 27-03-2018 |
| | | | CN | 115005830 A | 06-09-2022 |
| | | | US | 2018277243 A1 | 27-09-2018 |
| | | | WO | 2017059596 A1 | 13-04-2017 |
| US 2012278099 | A1 | 01-11-2012 | US | 2012278099 A1 | 01-11-2012 |
| | | | US | 2020357493 A1 | 12-11-2020 |
| US 2020121199 | A1 | 23-04-2020 | EP | 3870280 A1 | 01-09-2021 |
| | | | US | 2020121199 A1 | 23-04-2020 |
| | | | US | 2024252047 A1 | 01-08-2024 |
| | | | WO | 2020086528 A1 | 30-04-2020 |

EPO FORM P0459

For more details about this annex : see Official Journal of the European Patent Office, No. 12/82